(19) Europäisches Patentamt
European Patent Office
Office européen des brevets

(11) Veröffentlichungsnummer: **0 387 610 A2**

# (12) EUROPÄISCHE PATENTANMELDUNG

(21) Anmeldenummer: **90103936.2**

(22) Anmeldetag: **01.03.90**

(51) Int. Cl.5: **C07D 215/14, A61K 31/47**

(30) Priorität: **14.03.89 DE 3908298**

(43) Veröffentlichungstag der Anmeldung:
**19.09.90 Patentblatt 90/38**

(84) Benannte Vertragsstaaten:
**AT BE CH DE DK ES FR GB GR IT LI LU NL SE**

(71) Anmelder: **BAYER AG**
**D-5090 Leverkusen 1 Bayerwerk(DE)**

(72) Erfinder: **Raddatz, Siegfried, Dr.**
**Jakob-Böhme-Strasse 21**
**D-5000 Köln 80(DE)**
Erfinder: **Mohrs, Klaus, Dr.**
**Claudiusweg 9**
**D-5600 Wuppertal 1(DE)**
Erfinder: **Fruchtmann, Romanis**
**Konrad-Adenauer-Ufer 79**
**D-5000 Köln 1(DE)**
Erfinder: **Kohlsdorfer, Christian, Dr.**
**Franz-Stryck-Strasse 16**
**D-5042 Erftstadt(DE)**
Erfinder: **Müller-Peddinghaus, Reiner, Prof. Dr.**
**Klutstein 22a**
**D-5060 Bergisch Gladbach 2(DE)**
Erfinder: **Theisen-Popp, Pia, Dr.**
**Hermann-Löns-Strasse 64-66**
**D-5060 Bergisch Gladbach 2(DE)**

## (54) Substituierte (Chinolin-2-yl-methoxy)phenyl-N,N-sulfonylharnstoffe, Verfahren zu ihrer Herstellung und ihre Verwendung in Arzneimitteln.

(57) Substituierte (Chinolin-2-yl-methoxy)phenyl-N,N′-sulfonylharnstoffe können hergestellt werden durch Umsetzung von (Chinolin-2-yl-methoxy)-phenylaminen mit Sulfonylisocyanaten, oder durch Umsetzung von entsprechend geschützte Hydroxyanilinen mit Sulfonylisocyanaten und dann nach Abspaltung der Schutzgruppe mit Halogenmethylchinolinen, oder durch Umsetzung von (Chinolin-2-yl-methoxy)-phenyl-N,N′-sulfonylharnstoffe mit Sulfonsäurehalogeniden. Die substituierten (Chinolin-2-yl-methoxy)-phenyl-N,N′-sulfonylharnstoffe können als Wirkstoffe in Arzneimitteln eingesetzt werden, insbesondere als Lipoxygenaseinhibitoren.

EP 0 387 610 A2

**Substituierte (Chinolin-2-yl-methoxy)phenyl-N,N′-sulfonylharnstoffe, Verfahren zu ihrer Herstellung und ihre Verwendung in Arzneimitteln**

Die Erfindung betrifft substituierte (Chinolin-2-yl-methoxy)phenyl-N,N′-sulfonylharnstoffe, Verfahren zu ihrer Herstellung und ihre Verwendung in Arzneimitteln.

Es ist bekannt, daß Sulfonylharnstoffe eine antidiabetische Wirkung besitzen (vgl. W. Forth, Allgemeine und Spezielle Pharmakologie und Toxikologie, 4. Aufl., 1983, B.I.-Wissenschaftsverlag). Ferner sind N,N-Dimethyl-N′-[3-(2-chinolyl-methoxy)phenyl-harnstoffe in JP 82 64 675, Appl. 80/140 091 beschrieben worden.

Außerdem ist bekannt, daß substituierte (Chinolin-2-yl-methoxy)-phenylderivate eine lipoxygenasehemmende Wirkung zeigen (EP 110 405).

Es wurden nun substituierte (Chinolin-2-yl-methoxy)-N,N′-sulfonylharnstoffe der allgemeinen Formel (I)

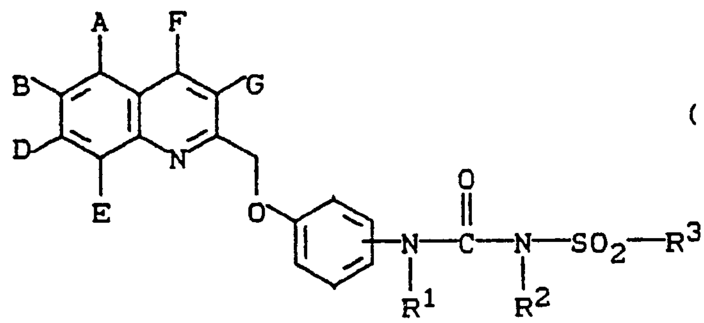

(I)

in welcher

A, B, D, E, F und G gleich oder verschieden sind und

- für Wasserstoff, Hydroxy, Halogen, Carboxy, Nitro, Trifluormethyl, Trifluormethoxy oder eine Gruppe der Formel $-NR^4R^5$ stehen,

worin

$R^4$ und $R^5$ gleich oder verschieden sind und Wasserstoff, geradkettiges oder verzweigtes Alkyl mit bis zu 8 Kohlenstoffatomen oder Aryl mit 6 bis 10 Kohlenstoffatomen bedeuten,

- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel $-NR^4R^5$ substituiert ist,

worin

$R^4$ und $R^5$ die oben angegebene Bedeutung haben,

- für Aryl mit 6 bis 10 Kohlenstoffatomen stehen, das gegebenenfalls durch Halogen, Hydroxy, Nitro, Cyano, geradkettiges oder verzweigtes Alkyl, Alkoxy oder Alkoxycarbonyl mit jeweils bis zu 8 Kohlenstoffatomen oder durch eine Gruppe der Formel $-NR^4R^5$ substituiert ist,

worin

$R^4$ und $R^5$ die oben angegebene Bedeutung haben,

$R^1$ und $R^2$ gleich oder verschieden sind und

- für Wasserstoff, oder
- für Cycloalkyl mit 3 bis 8 Kohlenstoffatomen stehen,
- für geradkettiges oder verzweigtes Alkyl mit bis zu 12 Kohlenstoffatomen stehen, das gegebenenfalls durch Halogen, Nitro, Cyano, Hydroxy, Trifluormethyl, Phenyl, Cycloalkyl mit 3 bis 8 Kohlenstoffatomen oder durch eine Gruppe der Formel $-NR^4R^5$ substituiert ist,

worin

$R^4$ und $R^5$ die oben angegebene Bedeutung haben,

$R^3$ die oben angegebene Bedeutung von $R^1$ und $R^2$ hat und mit dieser gleich oder verschieden ist, oder

- für einen 5- bis 7-gliedrigen Heterocyclus mit bis zu 4 verschiedenen Heteroatomen aus der Reihe Schwefel, Sauerstoff oder Stickstoff, oder für Aryl mit 6 bis 10 Kohlenstoffatomen steht, wobei der Heterocyclus und der Arylrest gegebenenfalls bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl, Trifluormethoxy oder durch eine Gruppe der Formel $-NR^4R^5$ substituiert sind,

wobei

$R^4$ und $R^5$ die oben angegebene Bedeutung haben
und deren Salze gefunden.

Im Rahmen der vorliegenden Erfindung werden physiologisch unbedenkliche Salze bevorzugt. Physiologisch unbedenkliche Salze der substituierten (Chinolin-2-yl-methoxy)phenyl-N,N′-sulfonylharnstoffe können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Die erfindungsgemäßen Verbindungen der allgemeinen Formel (I) zeigen überraschenderweise eine hohe in vitro Aktivität als Leukotrienhemmer und eine starke in vivo Wirkung nach oraler Applikation.

Bevorzugt sind Verbindungen der allgemeinen Formel (I),
in welcher
A, B, D, E, F und G gleich oder verschieden sind und
- für Wasserstoff, Hydroxy, Fluor, Chlor, Brom, Carboxy, Nitro, Trifluormethyl, Trifluormethoxy oder eine Gruppe der Formel $-NR^4R^5$ stehen,
worin
$R^4$ und $R^5$ gleich oder verschieden sind und Wasserstoff, geradkettiges oder verzweigtes Alkyl mit bis zu 6 Kohlenstoffatomen oder Phenyl bedeuten,
- für geradkettiges oder verzweigtes Alkyl, Alkoxy oder Alkoxycarbonyl mit jeweils bis zu 10 Kohlenstoffatomen stehen, das gegebenenfalls durch Hydroxy, Fluor, Chlor, Brom, Nitro, Cyano oder eine Gruppe der Formel $-NR^4R^5$ substituiert ist,
worin
$R^4$ und $R^5$ die oben angegebene Bedeutung haben,
- für Phenyl stehen, das gegebenenfalls durch Fluor, Chlor, Brom, Hydroxy, Nitro, Cyano, geradkettiges oder verzweigtes Alkyl, Alkoxy oder Alkoxycarbonyl mit jeweils bis zu 6 Kohlenstoffatomen oder durch eine Gruppe der Formel $-NR^4R^5$ substituiert ist,
worin
$R^4$ und $R^5$ die oben angegebene Bedeutung haben,
$R^1$ und $R^2$ gleich oder verschieden sind und
- für Wasserstoff oder
- für Cyclopropyl, Cyclobutyl, Cyclopentyl, Cyclohexyl oder Cycloheptyl stehen,
- für geradkettiges oder verzweigtes Alkyl mit bis zu 8 Kohlenstoffatomen stehen, das gegebenenfalls durch Fluor, Chlor, Brom, Nitro, Cyano, Hydroxy, Trifluormethyl, Phenyl, Cyclopropyl, Cyclobutyl, Cyclopentyl, Cyclohexyl, Cycloheptyl oder durch eine Gruppe der Formel $-NR^4R^5$ substituiert ist,
worin
$R^4$ und $R^5$ die oben angegebene Bedeutung haben,
$R^3$ die oben angegebene Bedeutung von $R^1$ und $R^2$ hat und mit dieser gleich oder verschieden ist, oder
- für Thienyl, Furanyl, Pyrryl, Pyrimidyl, Pyridyl oder Phenyl steht, die gegebenenfalls bis zu 2-fach gleich oder verschieden durch Fluor, Chlor, Brom, Nitro, Cyano, Hydroxy, geradkettiges oder verzweigtes Alkyl, Alkylthio oder Alkoxy mit bis zu 6 Kohlenstoffatomen, Trifluormethyl, Trifluormethoxy oder durch eine Gruppe der Formel $-NR^4R^5$ substituiert sind
und deren Salze.

Besonders bevorzugt sind Verbindungen der allgemeinen Formel (I)
in welcher
A, B, D, E, F und G gleich oder verschieden sind und
- für Wasserstoff, Fluor, Chlor, Brom, Nitro oder Trifluormethyl stehen,
- für Methyl, Ethyl, Propyl, Isopropyl, Butyl oder tert.Butyl stehen,
$R^1$ und $R^2$ gleich oder verschieden sind und
- für Wasserstoff oder
- für Cyclopropyl, Cyclopentyl oder Cyclohexyl stehen,
- für geradkettiges oder verzweigtes Alkyl mit bis zu 6 Kohlenstoffatomen stehen, das gegebenenfalls durch Fluor, Chlor, Brom, Phenyl, Cyclopentyl, Cyclohexyl oder Cycloheptyl substituiert ist,
$R^3$ die oben angegebene Bedeutung von $R^1$ und $R^2$ hat und mit dieser gleich oder verschieden ist, oder
- für Phenyl steht, das gegebenenfalls durch Fluor, Chlor, Brom oder geradkettiges oder verzweigtes Alkyl mit bis zu 4 Kohlenstoffatomen substituiert ist

und deren Salze.

Ganz besonders bevorzugt sind Verbindungen der allgemeinen Formel I, in denen die Chinolylmethoxygruppierung am Phenyl in 4-Stellung zur Sulfonylharnstoffgruppe steht.

Als ganz besonders bevorzugt werden Verbindungen der allgemeinen Formel (I) ausgewählt, in welchen
A, B, D E, F und G für Wasserstoff stehen,
$R^2$ und $R^3$ die oben angegebene Bedeutung haben und
$R^1$ für Cyclopentyl oder Methylcyclohexyl steht und
in welchen
A, B, D, E, F, G und $R^2$ die oben angegebene Bedeutung haben,
$R^1$ für Wasserstoff, n-Pentyl oder Cyclopentyl steht
und
$R^3$ für Benzyl steht
und deren Salze.

Außerdem wurden Verfahren zur Herstellung der erfindungsgemäßen Verbindungen der allgemeinen Formel (I)

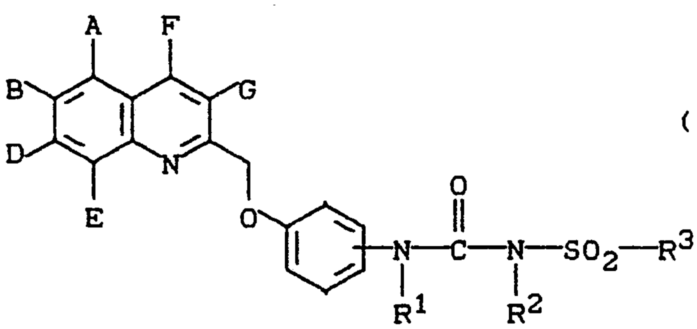

(I)

in welcher
A, B, D, E, F, G, $R^1$, $R^2$ und $R^3$ die oben angegebene Bedeutung haben,
gefunden, die dadurch gekennzeichnet sind, daß man

[A] Verbindungen der allgemeinen Formel (II),

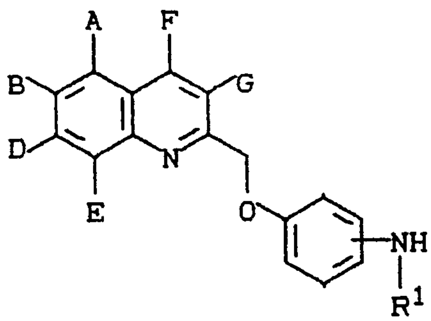

(II)

in welcher
A, B, D, E, F, G und $R^1$ die oben angegebene Bedeutung haben,
mit Sulfonylisocyanaten der allgemeinen Formel (III)
$R^3$-$SO_2$-NCO (III)
in welcher
$R^3$ die oben angegebene Bedeutung hat,
in einem inerten Lösemittel zu Verbindungen der allgemeinen Formel (Ia)

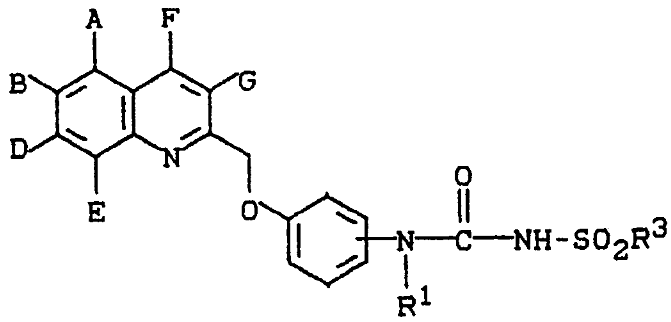

(Ia)

in welcher
A, B, D, E, F, G, $R^1$ und $R^3$ die oben angegebene Bedeutung haben,
umsetzt,
und im Fall der Verbindungen der Formel (I) mit $R^2 \neq H$ anschließend die Verbindungen der Formel (Ia) mit Alkylierungsmitteln in inerten Lösemitteln alkyliert,
oder indem man
[B] Verbindungen der allgemeinen Formel (IV)

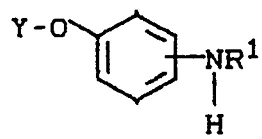

(IV)

in welcher
$R^1$ die oben angegebene Bedeutung hat
und
Y - für eine typische Hydroxyschutzgruppe, wie beispielsweise Benzyl oder tert.Butyl steht,
zunächst mit Verbindungen der allgemeinen Formel (III) in inerten Lösemitteln zu Verbindungen der allgemeinen Formel (V)

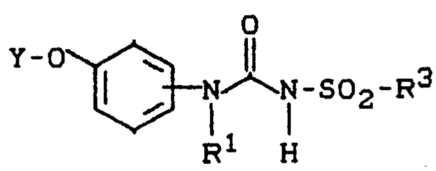

(V)

worin
$R^1$, $R^3$ und Y die oben angegebene Bedeutung haben,
umsetzt,
dann die Schutzgruppe Y nach üblicher Methode abspaltet
und anschließend mit Halogenmethylchinolinen der Formel (VI)

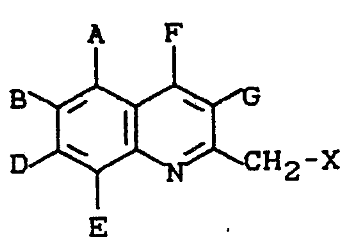

(VI)

in welcher
A, B, D, E, F und G die oben angegebene Bedeutung haben
und
X - für Halogen steht,
verethert,
und im Fall der Verbindungen der Formel (I), mit $R^2 \neq H$ anschließend mit Alkylierungsmitteln in inerten Lösemitteln alkyliert,
oder indem man
[C] Verbindungen der allgemeinen Formel (VII)

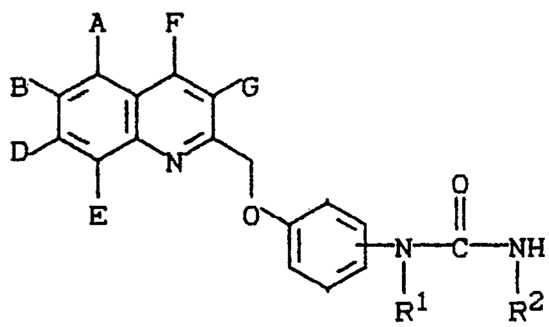

(VII)

in welcher
A, B, D, E, F, G, $R^1$ und $R^2$ die oben angegebene Bedeutung haben,
mit Sulfonsäurehalogeniden der allgemeinen Formel (VIII)
$R^3$-$SO_2$-X (VIII)
in welcher
$R^3$ und X die oben angegebene Bedeutung haben,
in inerten Lösemitteln, gegebenenfalls in Anwesenheit von Basen umsetzt.

Die erfindungsgemäßen Verfahren können an folgenden Formelschemata erläutert werden:

[A]

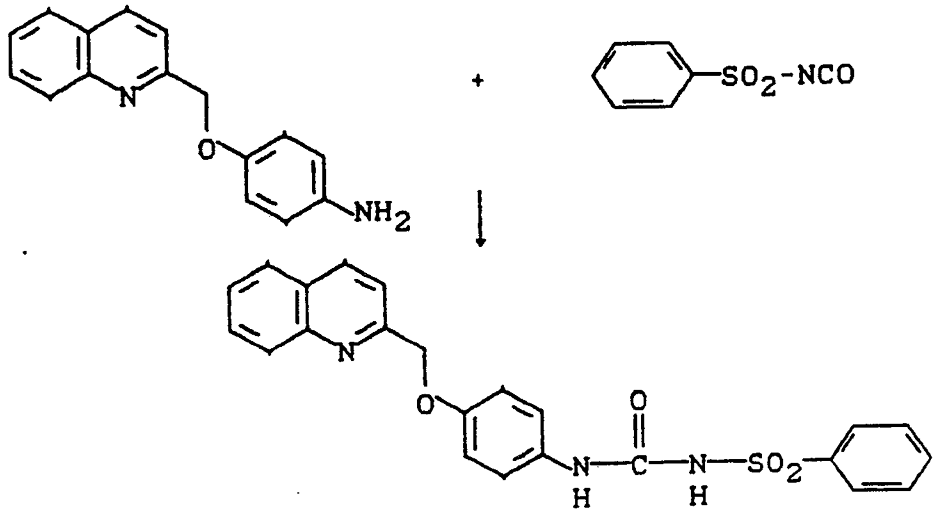

[B]

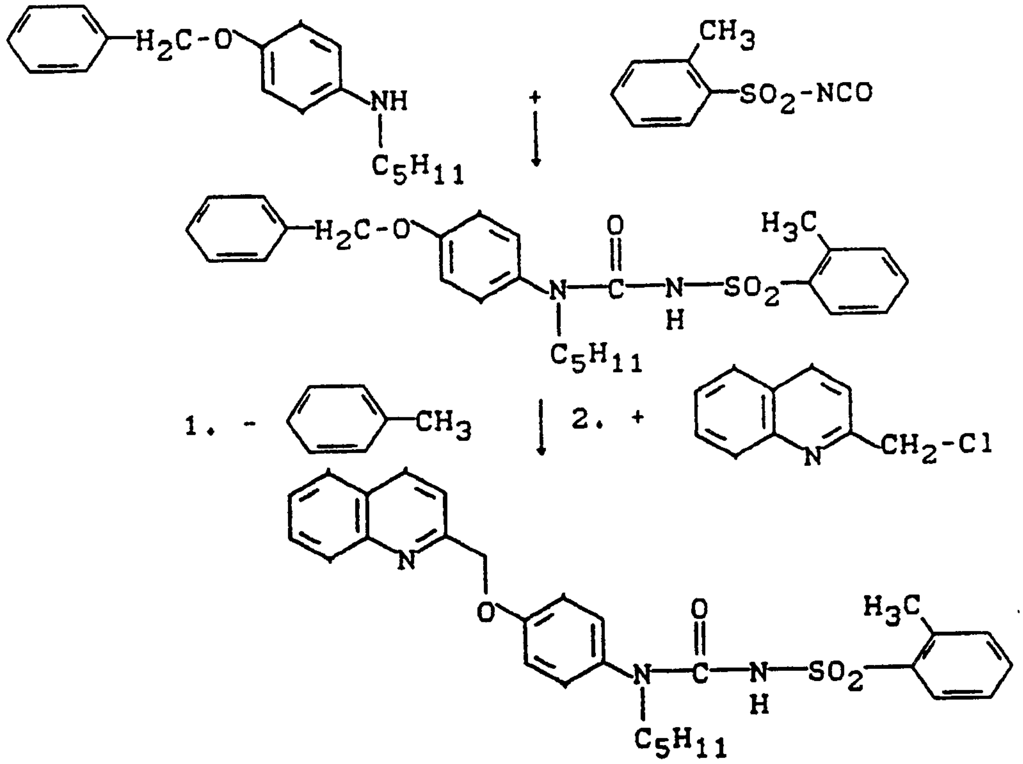

[C]

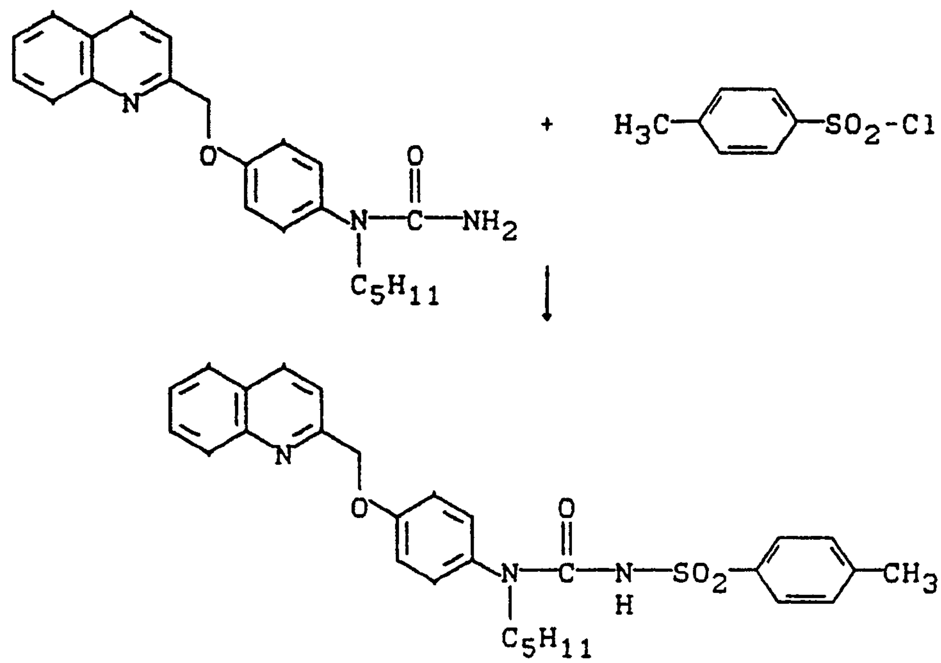

Als Lösemittel für das erfindungsgemäße Verfahren [A] eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Das erfindungsgemäße Verfahren [A] wird im allgemeinen in einem Temperaturbereich von -80 °C bis +80 °C, bevorzugt von -80 °C bis 0 °C durchgeführt.

Das erfindungsgemäße Verfahren [A] wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 3 Mol, bevorzugt 1 bis 2 Mol, besonders bevorzugt 1 Mol Sulfonylisocyanat, bezogen auf 1 Mol des Amins ein.

Die Sulfonylisocyanate der allgemeinen Formel (III) sind bekannt oder können nach bekannten Methoden hergestellt werden [C. King, J. Org. Chem. 25, 352 (1960); F. Effenberger, R. Gleiter, Chem. Ber. 97, 1576 (1964); H. Ulrich, A.A.R. Sayigh, Angew. Chem. 78, 761 (1966); Houben-Weyl VIII, 128].

Die Verbindungen der allgemeinen Formel (II) sind an sich bekannt oder können nach üblicher Methode hergestellt werden [vgl. Ger. Offen. DE 36 07 382].

Als Lösemittel für alle Teilschritte des Verfahrens [B] können die für Verfahren [A] aufgeführten Lösemittel benutzt werden.

Für die Umsetzung mit Sulfonylisocyanaten gelten die unter Verfahren [A] aufgeführten Bedingungen.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley & Sons, 1981, New York].

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu

gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhdyrid, einzusetzen.

Die Veretherung erfolgt im allgemeinen in einem Temperaturbereich von 0°C bis +150°C, bevorzugt von +10°C bis +100°C.

Die Veretherung wird wie alle anderen Teilschritte des erfindungsgemäßen Verfahrens [B] im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol des Reaktionspartners ein. Die Base wird im allgemeinen in einer Menge von 0,5 bis 5 Mol, bevorzugt von 1 bis 3 Mol bezogen auf das Halogenid eingesetzt.

Die Verbindungen der allgemeinen Formel (IV) und (V) sind an sich bekannt oder können nach üblicher Methode hergestellt werden [vgl. A. Ulrich, B. Tucker, A.A. R. Sayigh, J. Org. Chem. 31, 2658 (1966).

Ebenfalls sind die Verbindungen der allgemeinen Formel (VI) und ihre Darstellung bekannt.

Beispielsweise können folgende Halogenide erfindungsgemäß verwendet werden:

8-Chlor-2-chlormethyl-chinolin
7-Chlor-2-chlormethyl-chinolin
6-Fluor-2-Chlormethyl-chinolin

Als Lösemittel für das Verfahren [C] können die unter Verfahren [A] aufgeführten Lösemittel verwendet werden.

Basen für das erfindungsgemäße Verfahren [C] können übliche basische Verbindungen sein. Hierzu gehören vorzugsweise Alkali- oder Erdalkalihydroxide wie Lithiumhydroxid, Natriumhydroxid, Kaliumhydroxid oder Bariumhydroxid, oder Alkalihydride wie Natriumhydrid, oder Alkali- oder Erdalkalicarbonate wie Natriumcarbonat, Natriumhydrogencarbonat, Kaliumcarbonat oder Calciumcarbonat, oder Alkalialkoholate wie beispielsweise Natriummethanolat, Natriumethanolat, Kaliummethanolat, Kaliumethanolat oder Kalium-tert.-butylat, oder Alkaliamide wie Natriumamid oder Lithiumdiisopropylamid, oder organische Amine wie Benzyltrimethylammoniumhydroxid, Tetrabutylammoniumhydroxid, Pyridin, Dimethylaminopyridin, Triethylamin, N-Methylpiperidin, 1,5-Diazabicyclo[4,3,0]non-5-en oder 1,5-Diazabicyclo[5,4,0]undec-5-en.

Das erfindungsgemäße Verfahren [C] wird im allgemeinen in einem Temperaturbereich von -30°C bis +150°C, bevorzugt von -20°C bis +80°C, durchgeführt.

Das erfindungsgemäße Verfahren [C] wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 5 Mol, bevorzugt 1 bis 2 Mol, besonders bevorzugt 1 Mol Sulfonsäurehalogenid, bezogen auf 1 Mol des Amins, ein. Die Base wird im allgemeinen in einer Menge von 1 bis 10 Mol, bevorzugt von 1 bis 5 Mol, bezogen auf das Sulfonsäurehalogenid, eingesetzt.

Die Verbindungen der allgemeinen Formel (VII) sind an sich bekannt oder können nach üblicher Methode hergestellt werden [vgl. JP 82 64 675; Appl. 80/140.091].

Die Sulfonsäurehalogenide der allgemeinen Formel (VIII) sind bekannt [vgl. R.V. Vitzgert, Uspekhi, Khimii 32, 3 (1963); Russian Chem. Rev. 32, 1 (1963)].

Als Sulfonsäurehalogenide für das erfindungsgemäße Verfahren [C] seien beispielsweise genannt:

4-Phenyl-sulfonylchlorid
4-Toluol-sulfonylchlorid
4-Chlorphenyl-sulfonylchlorid
4-Methoxyphenyl-sulfonylchlorid
Propyl-sulfonylchlorid
Butyl-sulfonylchlorid
Isobutyl-sulfonylchlorid

Als Alkylierungsmittel bei dem Verfahren [A] und [B] können beispielsweise ($C_1$-$C_8$)-Alkylhalogenide, Sulfonsäureester oder substituierte oder unsubstituierte ($C_1$-$C_6$)-Dialkyl- oder ($C_6$-$C_{10}$)-Diarylsulfate, vorzugsweise Methyljodid, p-Toluolsulfonsäureester oder Dimethylsulfat eingesetzt werden.

Die Alkylierung wird in den oben aufgeführten Lösemitteln bei Temperaturen von 0°C bis +150°C, vorzugsweise bei Raumtemperaturen bis +100°C bei Normaldruck durchgeführt.

Die erfindungsgemäßen substituierten 3- oder 4-(Chinolyl-2-yl-methoxy)phenyl-N,N'-sulfonylharnstoffe können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen substituierten (Chinolyl-2-yl-methoxy)phenyl-N,N′-sulfonylharnstoffe können sowohl in der Humanmedizin als auch in der Veterinärmedizin angewendet werden.

Die pharmakologischen Wirkdaten der erfindungsgemäßen Substanzen werden durch folgende Methode bestimmt:

Als Maß für die Lipoxygenase-Hemmung wurde die Freisetzung von Leukotrien $B_4$ ($LTB_4$) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148 - 2152 (1979), bestimmt. Die in vivo-Aktivität der Verbindungen wurde mit dem Mäuseohr-Entzündungsmodell nach Young, J.M. et al., J. of Investigative Dermatology 82, 367 - 371, (1984) nachgewiesen.

In der Tabelle 1 sind beispielhaft die nach diesem Test erzielten Werte einiger erfindungsgemäßer Verbindungen aufgeführt:

Tabelle 1

| Beispiel | LO-Hemmung $IC_{50}$ (µM) |
|---|---|
| 3 | 0.036 |
| 4 | 0,033 |
| 5 | 0,41 |
| 6 | 0,36 |
| 7 | 0,24 |
| 8 | 0,29 |

Die neuen Wirkstoffe können in an sich bekannter Weise unter Verwendung inerter nichttoxischer, pharmazeutisch geeigneter Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungs mittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Eli xieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

Herstellungsbeispiele

Beispiel 1

2-(4-Aminophenoxymethyl)chinolin

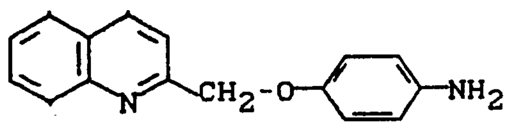

117,8 g (0,42 mol) 2-(4-Nitrophenoxymethyl)chinolin wurden in 1 l Methanol/Tetrahydrofuran (1:1) gelöst. Anschließend wurden ca. 5 g Raney-Nickel zugegeben und auf 35 °C erwärmt. Dann wurden 63.1 g (1,26 mol) Hydrazinhydrat x $H_2O$ zugetropft und über Nacht gerührt. Es wurde vom Rückstand abfiltriert, die Lösung im Vakuum eingeengt und der Rückstand mit Methylenchlorid aufgenommen. Danach wurde mit Wasser gewaschen und die organische Phase mit konz. Salzsäure versetzt. Der ausgefallene Niederschlag wurde abfiltriert, mit 2 N Salzsäure gewaschen, in Wasser gelöst und mit 20%iger NaOH alkalisiert. Der Rückstand wurde im Vakuum getrocknet.

Ausbeute: 88,0 g (92,1% der Theorie)

Fp. = 131 °C

Beispiel 2

2-(4-n-Pentylaminophenoxymethyl)chinolin

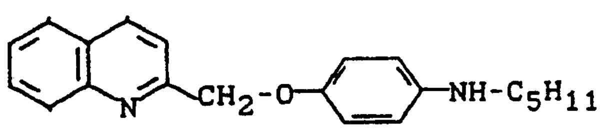

12,5 g (0,05 mol) der Verbindung aus Beispiel 1 wurden in Methanol gelöst. Danach wurden 2,0 g (0,03 mol) Natriumcyanoborhydrid portionsweise zugegeben. Zu dieser Lösung wurden 5,3 ml (0,05 mol) Pentanal vorsichtig zugetropft. Nach 48 h Rühren bei Raumtemperatur wurde die Lösung im Vakuum konzentriert, der Rückstand in Methylenchlorid gelöst und mit 2 N Salzsäure gewaschen. Die organische Phase wurde abgetrennt, getrocknet, im Vakuum konzentriert und über eine 1000 $cm^3$ Kieselgel-60-Säule

gegeben. Als Laufmittel wurde das System Methylenchlorid/Essigester 7:3 eingesetzt.
Ausbeute: 5,5 g (34,4% der Theorie)
Fp. = 54 - 55° C

Beispiel 3

N-(4-methylphenylsulfonyl)-N′-pentyl-N′-4-(2-chinolylmethyloxy)phenylharnstoff

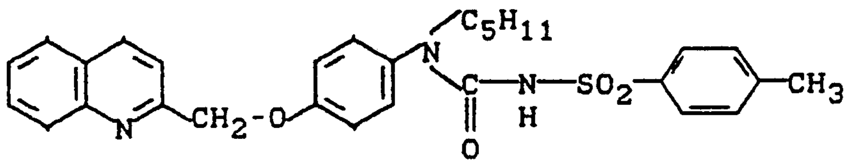

1,9 g (0,006 mol) der Verbindung aus Beispiel 2 wurden in 50 ml Methylenchlorid gelöst und auf -78° C abge kühlt. Danach wurde die Apparatur mehrfach mit Argon gespült. Unter Argon wurden anschließend 1,18 g (0,006 mol) p-Tolylsulfonylisocyanat zugetropft. Nach einer Stunde Rühren bei -78° C wurde auf Raumtemperatur erwärmt und im Vakuum eingeengt. Das Öl wurde mit Ether suspendiert, zunächst auf Raumtemperatur erhitzt und anschließend abgekühlt. Dabei kristallisiert das Produkt aus. Der Rückstand wurde abgesaugt und im Vakuum getrocknet.
Ausbeute: 2,6 g (83,8% der Theorie)
Fp. = 157° C

Beispiel 4

N-(2-methylphenylsulfonyl)-N′-pentyl-N′-4-(2-chinolylmethyloxy)phenyl-harnstoff

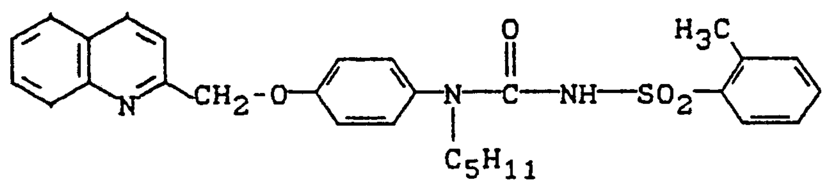

Die Umsetzung erfolgte in Analogie zu der Vorschrift für Beispiel 3 aus 3,0 g (0,0094 mol) der Verbindung aus Beispiel 1 und 1,8 g (0,0094 mol) o-Tolylsulfonylisocyanat.
Ausbeute: 4,3 g (88,5% der Theorie)
Fp. = 174° C

Beispiel 5

N-(2-methylphenylsulfonyl)-N′-4-(2-chinolylmethoxy)phenyl-harnstoff

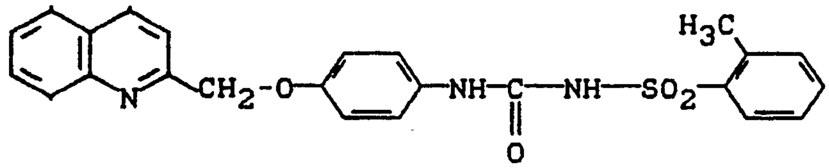

In Analogie zu der Vorschrift für Beispiel 3 wurden 6,25 g (0,025 mol) der Verbindung aus Beispiel 1 und 5,1 g (0,025 mol) o-Tolylsulfonylisocyanat umgesetzt.
Ausbeute: 11,0 g (98,4% der Theorie)
Fp. = 142° C

Beispiel 6

N-(4-methylphenylsulfonyl)-N′-4-(2-chinolylmethoxy)phenyl-harnstoff

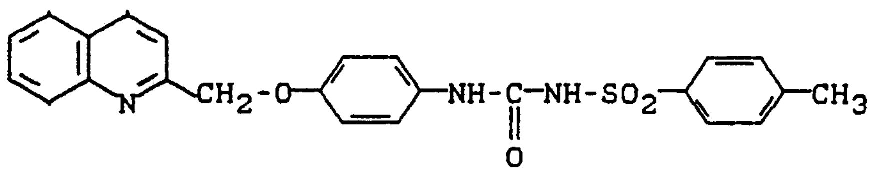

In Analogie zu der Vorschrift für Beispiel 3 wurden 6,25 g (0,025 mol) der Verbindung aus Beispiel 1 mit 4,9 g (0,025 mol) p-Tolylsulfonylisocyanat umgesetzt.
Ausbeute: 11,1 g (99,3% der Theorie)
Fp. = 168° C

Analog den Vorschriften für die Beispiele 5 und 6 können die in der folgenden Tabelle aufgeführten Verbindungen hergestellt werden:

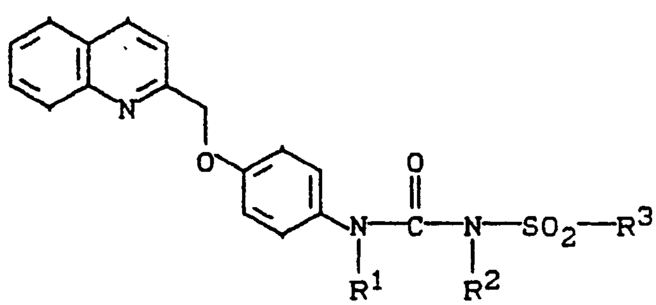

| Beispiel-Nr. | $R^1$ | $R^2$ | $R^3$ | Fp.: |
|---|---|---|---|---|
| 7 | 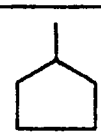 | H | 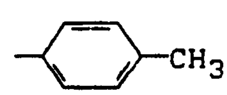 | 147° C (Zers.) |
| 8 | 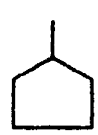 | H | 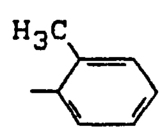 | 178° C (Zers.) |

Beispiel 9

N-Methansulfonyl-N′-4-(2-chinolinylmethoxy)-phenylharnstoff

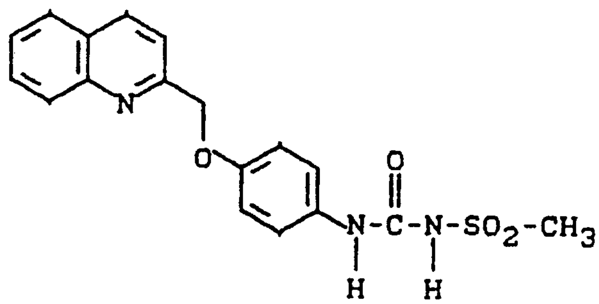

Die Titelverbindung wurde analog der Vorschrift des Beispiels 3 aus 2,5 g (0,01 mol) der Verbindung aus Beispiel 1 und 1,2 g (0,01 mol) Methansulfonylisocyanat hergestellt.
Farblose Kristalle, Fp.: 187° C (Zers.)
Ausbeute: 3,5 g (= 94,3 % der Theorie).

Beispiel 10

N-Benzylsulfonyl-N′-4-(2-chinolinylmethoxy)-phenylharnstoff

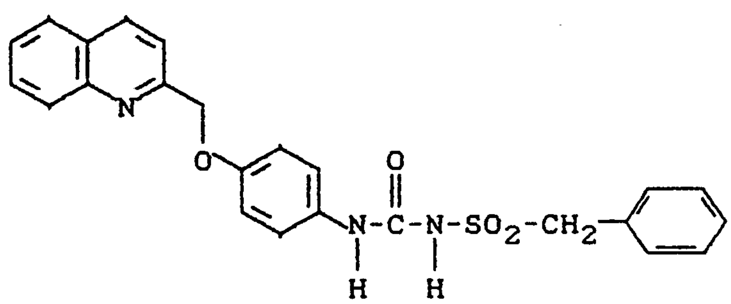

Die Titelverbindung wurde in Analogie zu Beispiel 3 aus 2,5 g (0,01 mol) der Verbindung aus Beispiel 1 und 2,0 g (0,01 mol) Benzylsulfonylisocyanat hergestellt.
Farblose Kristalle, Fp.: 183° C (Zers.)
Ausbeute: 3,5 g (= 78,2 % der Theorie).

Beispiel 11

N-Mesyl-N′-n-pentyl-N′-4-(2-chinolinylmethoxy)-phenylharnstoff

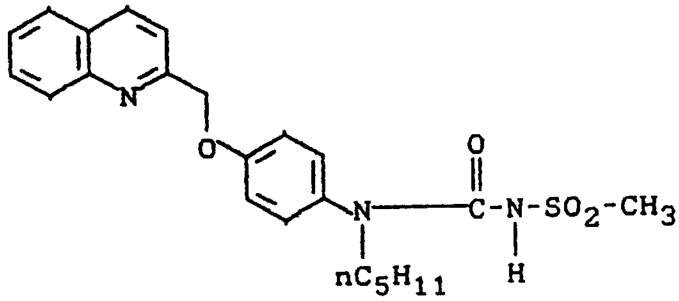

Analog der Vorschrift für Beispiel 3 wurde die Titelverbindung aus 1,92 g (0,006 mol) der Verbindung aus Beispiel 2 und 0,73 g (0,006 mol) Methansulfonylisocyanat hergestellt.
Farblose, glasartige Substanz,
Ausbeute: 1,4 g (52,9 % der Theorie).

Beispiel 12

N-Benzylsulfonyl-N′-n-pentyl-N′-4-(2-chinolinylmethoxy)-harnstoff

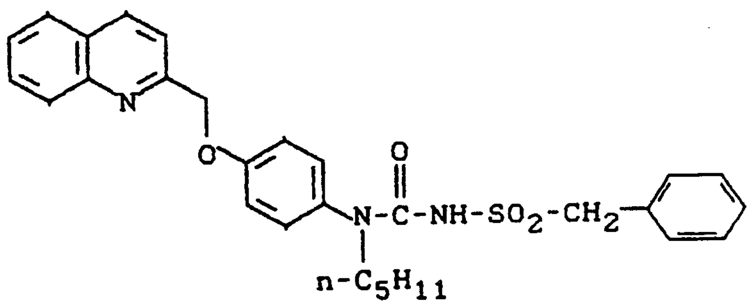

Analog der Vorschrift für Beispiel 3 wurde die Titelverbindung aus 2,8 g (0,0088 mol) der Verbindung aus Beispiel 2 und 2,2 g (0,0088 mol) Benzylsulfonylisocyanat hergestellt.
Farblose, glasartige Substanz,
Ausbeute: 1,9 g (= 41,7 % der Theorie).

Beispiel 13

2-(4-Cyclohexyl-methyl-amino-phenoxymethyl)-chinolin

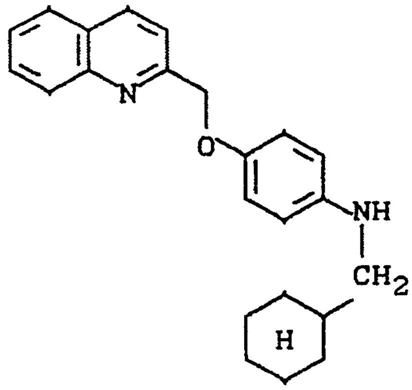

10 g (0,04 mol) der Verbindung aus Beispiel 1, 7,1 g (0,04 mol) Cyclohexyl-methyl-bromid, 8,1 g (0,08 mol) Triethylamin und 5,6 g (0,04 mol) Kaliumcarbonat werden in 40 ml Dimethylformamid (getrocknet) unter Argonatmosphäre über Nacht bei 60-70° C gerührt. Nach dem Abkühlen wird im Vakuum zur Trockne eingeengt, in Dichlormethan aufgenommen und zweimal mit 1n Natronlauge ausgeschüttelt. Nach Neutralwaschen und Trocknen mit Natriumsulfat wird im Vakuum auf ein kleines Volumen reduziert und der Rückstand säulenchromatographisch getrennt (Kieselgel 60, Dichlormethan/Essigsäureethylester = 20/1). Nach Einengen der geeigneten Fraktion wird der leicht gelbliche Rückstand aus n-Hexan kristallisiert.
Farblose Kristalle, Fp.: 94° C,
Ausbeute: 6,8 g (49,1 % der Theorie).

Beispiel 14

N-Mesyl-N′-cyclohexyl-methyl-N′-4-(2-chinolinylmethoxy)-phenylharnstoff

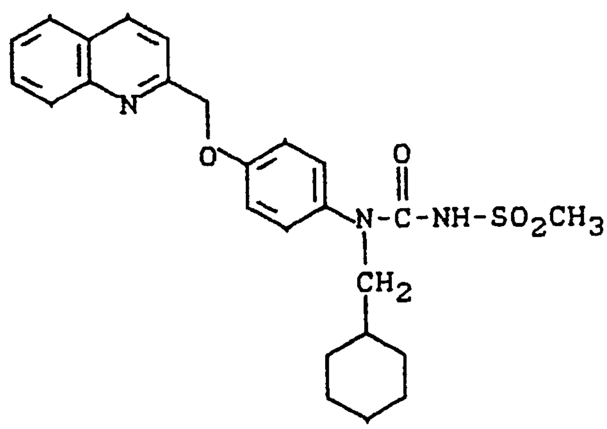

In Analogie zur Vorschrift für Beispiel 3 wurde die Titelverbindung aus 2,1 g (0,006 mol) 2-(4-Cyclohexyl-methyl-amino-phenoxymethyl)-chinolin (Beispiel 13) und 0,73 g (0,006 mol) Methansulfonylisocyanat hergestellt.
Farblose Kristalle, Fp.: 171° C,
Ausbeute: 2,5 g (89,1 % der Theorie).

Beispiel 15

2-(4-Cyclopentylaminophenoxymethyl)-chinolin

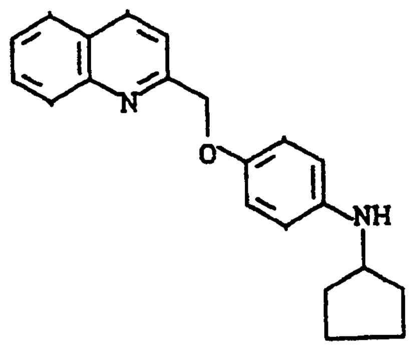

In Analogie zu Beispiel 13 wurde die Titelverbindung aus 10 g (0,04 mol) der Verbindung aus Beispiel 1 und 6 g (0,04 mol) Cyclopentylbromid bei einer Temperatur von +40 - +50 °C hergestellt.
Farblose Kristalle, Fp.: 88 °C,
Ausbeute: 0,6 g (51,9 % der Theorie).

Beispiel 16

N-Mesyl-N′-cyclopentyl-N′-4-(2-chinolinylmethoxy)-phenylharnstoff

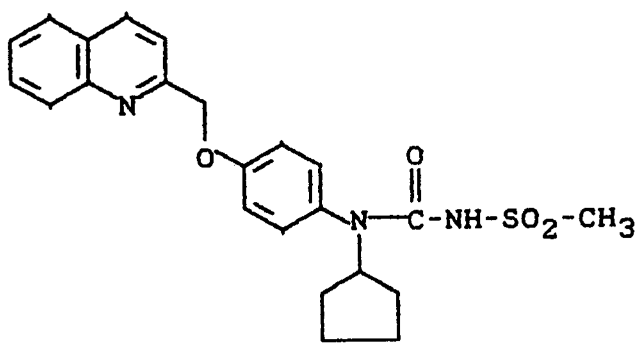

Die Titelverbindung wurde in Analogie zu Beispiel 3 aus 1,6 g (0,005 mol) der Verbindung aus Beispiel 15 und 0,6 g (0,005 mol) Methansulfonylisocyanat hergestellt.
Farblose Kristalle, Fp.: 175 °C (Zers.),
Ausbeute: 1,3 g (59,2 % der Theorie).

Beispiel 17

N-Benzylsulfonyl-N′-cyclopentyl-N′-4-(2-chinolinylmethoxy)-phenylharnstoff

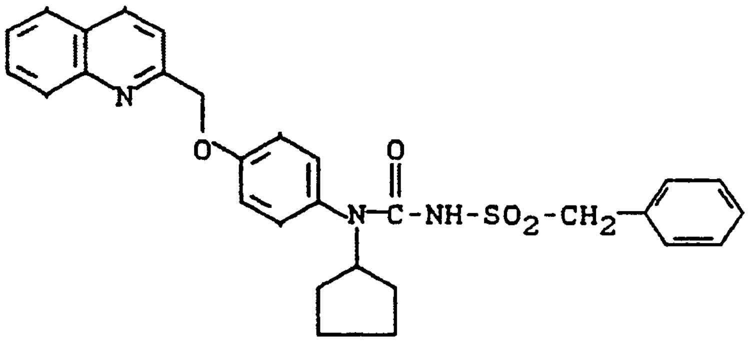

In Analogie zu Beispiel 3 wurde die Titelverbindung aus 3,2 g (0,01 mol) der Verbindung aus Beispiel 5 und 2,0 g (0,01 mol) Benzylsulfonylisocyanat hergestellt.
Farblose, glasartige Substanz,
Ausbeute: 1,1 g (21,3 % der Theorie).

Beispiel 18

N-Benzylsulfonyl-N′-4-(2-chinolinylmethoxy)-phenylharnstoff-Natriumsalz

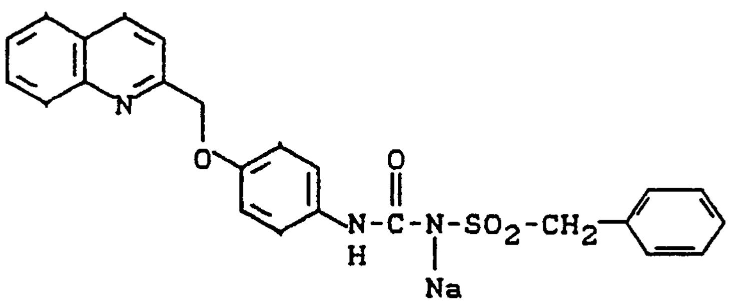

Man löste 1 g der Verbindung aus Beispiel 10 (0,0022 mol) in Ethanol/Tetrahydrofuran und versetzte mit der äquimolaren Menge 1n Natronlauge. Die Lösung wurde im Vakuum zur Trockene eingeengt und scharf getrocknet.
Farblose Substanz, Fp.: > 229° C (Zers.)

Beispiel 19

2-(3-Aminophenoxymethyl)-chinolin

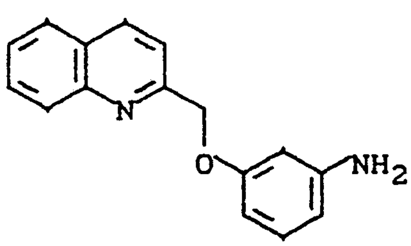

Die Verbindung wurde analog der Vorschrift für Beispiel 1 aus 50 g (0,178 mol) 2-(3-Nitrophenoxymethyl)-chinolin hergestellt. Die Rekristallisation erfolgte aus Diisopropylether.
Farblose Kristalle, Fp.: 100° C,
Ausbeute: 40,7 g (91,5 % der Theorie).

Beispiel 20

N-Tosyl-N′-3-(2-chinolinylmethoxy)-phenylharnstoff

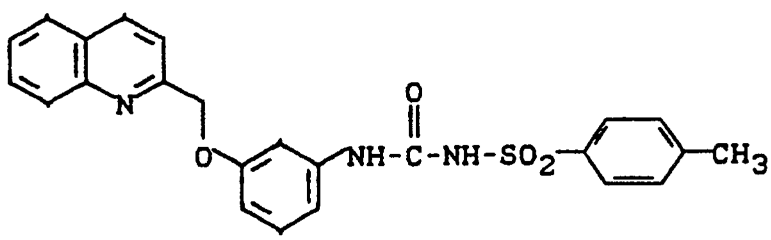

Die Titelverbindung wurde in Analogie zur Vorschrift für Beispiel 3 aus 2 g (0,008 mol) der Verbindung aus Beispiel 19 und 1,58 g (0,008 mol) 4-Toluolsulfonylisocyanat hergestellt.
Farblose Kristalle, Fp.: 121-122° C,
Ausbeute: 2,1 g (61,7 % der Theorie).

Beispiel 21

N-o-Tosyl-N′-3-(2-chinolinylmethoxy)-phenylharnstoff

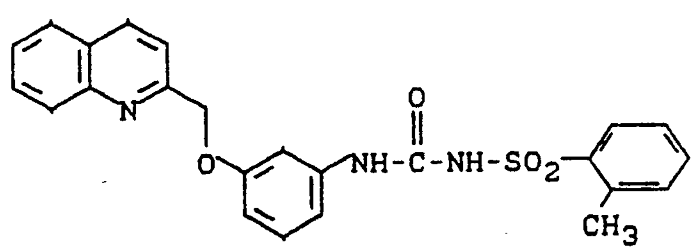

Analog der Vorschrift für Beispiel 3 wurde die Titelverbindung aus 2 g (0,008 mol) der Verbindung aus Beispiel 19 und 1,58 g (0,008 mol) 2-Toluolsulfonylisocyanat hergestellt.
Farblose Kristalle, Fp.: 125° C,
Ausbeute: 2,1 g (61,7 % der Theorie).

**Ansprüche**

1. Substituierte (Chinolin-2-yl-methoxy)phenyl-N,N′-sulfonylharnstoffe der allgemeinen Formel (I)

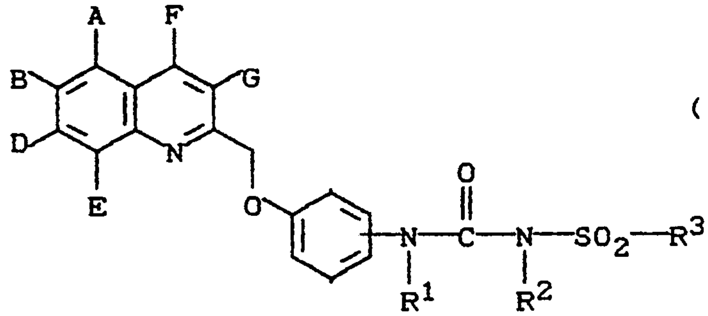

(I)

in welcher

A, B, D, E, F und G gleich oder verschieden sind und

- für Wasserstoff, Hydroxy, Halogen, Carboxy, Nitro, Trifluormethyl, Trifluormethoxy oder eine Gruppe der Formel $-NR^4R^5$ stehen,

worin

$R^4$ und $R^5$ gleich oder verschieden sind und Wasserstoff, geradkettiges oder verzweigtes Alkyl mit bis zu 8 Kohlenstoffatomen oder Aryl mit 6 bis 10 Kohlenstoffatomen bedeuten,

- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel $-NR^4R^5$ substituiert ist,

worin

$R^4$ und $R^5$ die oben angegebene Bedeutung haben,

- für Aryl mit 6 bis 10 Kohlenstoffatomen stehen, das gegebenenfalls durch Halogen, Hydroxy, Nitro, Cyano, geradkettiges oder verzweigtes Alkyl, Alkoxy oder Alkoxycarbonyl mit jeweils bis zu 8 Kohlenstoffatomen oder durch eine Gruppe der Formel $-NR^4R^5$ substituiert ist,

worin

$R^4$ und $R^5$ die oben angegebene Bedeutung haben,

$R^1$ und $R^2$ gleich oder verschieden sind und

- für Wasserstoff, oder

- für Cycloalkyl mit 3 bis 8 Kohlenstoffatomen stehen,

- für geradkettiges oder verzweigtes Alkyl mit bis zu 12 Kohlenstoffatomen stehen, das gegebenenfalls durch Halogen, Nitro, Cyano, Hydroxy, Trifluormethyl, Phenyl, Cycloalkyl mit 3 bis 8 Kohlenstoffatomen oder durch eine Gruppe der Formel $-NR^4R^5$ substituiert ist,

worin

$R^4$ und $R^5$ die oben angegebene Bedeutung haben,

$R^3$ die oben angegebene Bedeutung von $R^1$ und $R^2$ hat und mit dieser gleich oder verschieden ist, oder

- für einen 5- bis 7-gliedrigen Heterocyclus mit bis zu 4 verschiedenen Heteroatomen aus der Reihe Schwefel, Sauerstoff oder Stickstoff, oder für Aryl mit 6 bis 10 Kohlenstoffatomen steht, wobei der Heterocyclus und der Arylrest gegebenenfalls bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl, Trifluormethoxy oder durch eine Gruppe der Formel $-NR^4R^5$ substituiert sind,

wobei

$R^4$ und $R^5$ die oben angegebene Bedeutung haben

und deren Salze.

2. Substituierte (Chinolin-2-yl-methoxy)phenyl-N,N′-sulfonylharnstoffe der Formel (I) nach Anspruch 1

worin

A, B, D, E, F und G gleich oder verschieden sind und

- für Wasserstoff, Hydroxy, Fluor, Chlor, Brom, Carboxy, Nitro, Trifluormethyl, Trifluormethoxy oder eine Gruppe der Formel $-NR^4R^5$ stehen,

worin

$R^4$ und $R^5$ gleich oder verschieden sind und Wasserstoff, geradkettiges oder verzweigtes Alkyl mit bis zu 6 Kohlenstoffatomen oder Phenyl bedeuten,

- für geradkettiges oder verzweigtes Alkyl, Alkoxy oder Alkoxycarbonyl mit jeweils bis zu 10 Kohlenstoffatomen stehen, das gegebenenfalls durch Hydroxy, Fluor, Chlor, Brom, Nitro, Cyano oder eine Gruppe der Formel $-NR^4R^5$ substituiert ist,

worin

$R^4$ und $R^5$ die oben angegebene Bedeutung haben,
- für Phenyl stehen, das gegebenenfalls durch Fluor, Chlor, Brom, Hydroxy, Nitro, Cyano, geradkettiges oder verzweigtes Alkyl, Alkoxy oder Alkoxycarbonyl mit jeweils bis zu 6 Kohlenstoffatomen oder durch eine Gruppe der Formel $-NR^4R^5$ substituiert ist,
worin
$R^4$ und $R^5$ die oben angegebene Bedeutung haben,
$R^1$ und $R^2$ gleich oder verschieden sind und
- für Wasserstoff oder
- für Cyclopropyl, Cyclobutyl, Cyclopentyl, Cyclohexyl oder Cycloheptyl stehen,
- für geradkettiges oder verzweigtes Alkyl mit bis zu 8 Kohlenstoffatomen stehen, das gegebenenfalls durch Fluor, Chlor, Brom, Nitro, Cyano, Hydroxy, Trifluormethyl, Phenyl, Cyclopropyl, Cyclobutyl, Cyclopentyl, Cyclohexyl, Cycloheptyl oder durch eine Gruppe der Formel $-NR^4R^5$ substituiert ist,
worin
$R^4$ und $R^5$ die oben angegebene Bedeutung haben,
$R^3$ die oben angegebene Bedeutung von $R^1$ und $R^2$ hat und mit dieser gleich oder verschieden ist, oder
- für Thienyl, Furanyl, Pyrryl, Pyrimidyl, Pyridyl oder Phenyl steht, die gegebenenfalls bis zu 2-fach gleich oder verschieden durch Fluor, Chlor, Brom, Nitro, Cyano, Hydroxy, geradkettiges oder verzweigtes Alkyl, Alkylthio oder Alkoxy mit bis zu 6 Kohlenstoffatomen, Trifluormethyl, Trifluormethoxy oder durch eine Gruppe der Formel $-NR^4R^5$ substituiert sind
und deren Salze.

3. Substituierte (Chinolin-2-yl-methoxy)phenyl-N,N′-sulfonylharnstoffe der Formel (I), nach Anspruch 1
worin
A, B, D, E, F und G gleich oder verschieden sind und
- für Wasserstoff, Fluor, Chlor, Brom, Nitro oder Trifluormethyl stehen,
- für Methyl, Ethyl, Propyl, Isopropyl, Butyl oder tert.Butyl stehen,
$R^1$ und $R^2$ gleich oder verschieden sind und
- für Wasserstoff oder
- für Cyclopropyl, Cyclopentyl oder Cyclohexyl stehen,
- für geradkettiges oder verzweigtes Alkyl mit bis zu 6 Kohlenstoffatomen stehen, das gegebenenfalls durch Fluor, Chlor, Brom, Cyclopentyl, Cyclohexyl, Cycloheptyl oder Phenyl substituiert ist,
$R^3$ die oben angegebene Bedeutung von $R^1$ und $R^2$ hat und mit dieser gleich oder verschieden ist,
oder
- für Phenyl steht, das gegebenenfalls durch Fluor, Chlor, Brom oder geradkettiges oder verzweigtes Alkyl mit bis zu 4 Kohlenstoffatomen substituiert ist
und deren Salze.

4. Substituierte (Chinolin-2-yl-methoxy)phenyl-N,N′-sulfonylharnstoffe nach Anspruch 1 zur Behandlung von Krankheiten.

5. Verfahren zur Herstellung von substituierten (Chinolin-2-ylmethoxy)phenyl-N,N′-sulfonylharnstoffe der allgemeinen Formel (I)

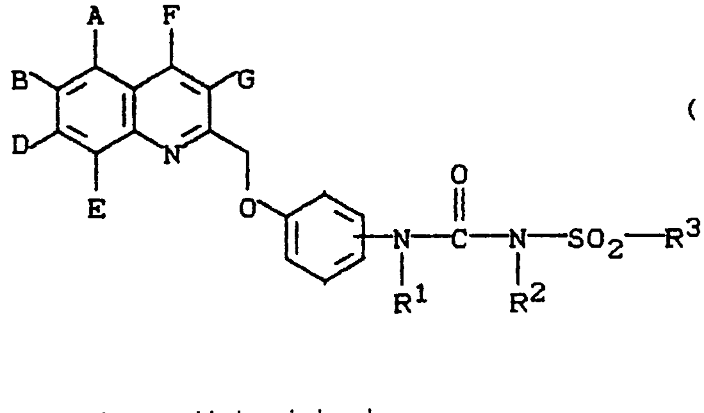

(I)

in welcher
A, B, D, E, F und G gleich oder verschieden sind und
- für Wasserstoff, Hydroxy, Halogen, Carboxy, Nitro, Trifluormethyl, Trifluormethoxy oder eine Gruppe der Formel $-NR^4R^5$ stehen,
worin
$R^4$ und $R^5$ gleich oder verschieden sind und Wasserstoff, geradkettiges oder verzweigtes Alkyl mit bis zu 8

Kohlenstoffatomen oder Aryl mit 6 bis 10 Kohlenstoffatomen bedeuten,
- für geradkettiges oder verzweigtes Alkyl, Alkoxy oder Alkoxycarbonyl mit jeweils bis zu 12 Kohlenstoffatomen stehen, das gegebenenfalls durch Hydroxy, Halogen, Nitro, Cyano oder eine Gruppe der Formel $-NR^4R^5$ substituiert ist,
worin
$R^4$ und $R^5$ die oben angegebene Bedeutung haben,
- für Aryl mit 6 bis 10 Kohlenstoffatomen stehen, das gegebenenfalls durch Halogen, Hydroxy, Nitro, Cyano, geradkettiges oder verzweigtes Alkyl, Alkoxy oder Alkoxycarbonyl mit jeweils bis zu 8 Kohlenstoffatomen oder durch eine Gruppe der Formel $-NR^4R^5$ substituiert ist,
worin
$R^4$ und $R^5$ die oben angegebene Bedeutung haben,
$R^1$ und $R^2$ gleich oder verschieden sind und
- für Wasserstoff, oder
- für Cycloalkyl mit 3 bis 8 Kohlenstoffatomen stehen,
- für geradkettiges oder verzweigtes Alkyl mit bis zu 12 Kohlenstoffatomen stehen, das gegebenenfalls durch Halogen, Nitro, Cyano, Hydroxy, Trifluormethyl, Phenyl, Cycloalkyl mit 3 bis 8 Kohlenstoffatomen oder durch eine Gruppe der Formel $-NR^4R^5$ substituiert ist,
worin
$R^4$ und $R^5$ die oben angegebene Bedeutung haben,
$R^3$ die oben angegebene Bedeutung von $R^1$ und $R^2$ hat und mit dieser gleich oder verschieden ist,
oder
- für einen 5- bis 7-gliedrigen Heterocyclus mit bis zu 4 verschiedenen Heteroatomen aus der Reihe Schwefel, Sauerstoff oder Stickstoff, oder für Aryl mit 6 bis 10 Kohlenstoffatomen steht, wobei der Heterocyclus und der Arylrest gegebenenfalls bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl, Trifluormethoxy oder durch eine Gruppe der Formel $-NR^4R^5$ substituiert sind,
wobei
$R^4$ und $R^5$ die oben angegebene Bedeutung haben
und deren Salze,
dadurch gekennzeichnet, daß man
[A] Verbindungen der allgemeinen Formel (II),

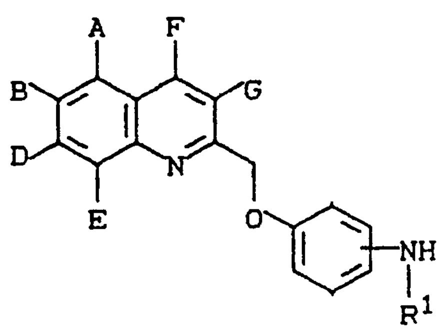

(II)

in welcher
A, B, D, E, F, G und $R^1$ die angegebene Bedeutung haben,
mit Sulfonylisocyanaten der allgemeinen Formel (III)
$R^3-SO_2-NCO$ (III)
in welcher
$R^3$ die oben angegebene Bedeutung hat,
in einem inerten Lösemittel zu Verbindungen der allgemeinen Formel (Ia)

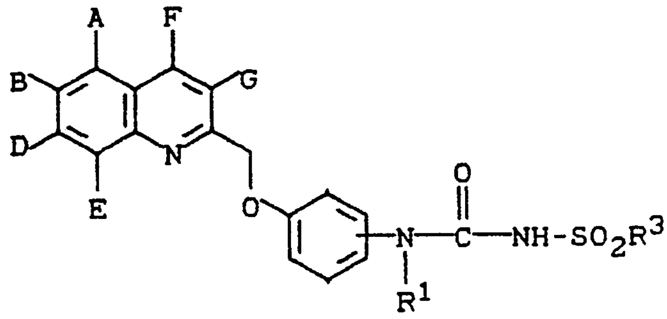

(Ia)

in welcher
A, B, D, E, F, G, $R^1$ und $R^3$ die oben angegebene Bedeutung haben,
umsetzt,
und im Fall der Verbindungen der Formel (I) mit $R^2 \neq H$ anschließend die Verbindungen der Formel (Ia) mit Alkylierungsmitteln in inerten Lösemitteln alkyliert,
oder indem man
[B] Verbindungen der allgemeinen Formel (IV)

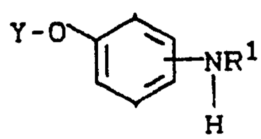

(IV)

in welcher
$R^1$ die oben angegebene Bedeutung hat
und
Y - für eine typische Hydroxyschutzgruppe, wie beispielsweise Benzyl oder tert.Butyl steht,
zunächst mit Verbindungen der allgemeinen Formel (III) in inerten Lösungsmitteln zu Verbindungen der allgemeinen Formel (V)

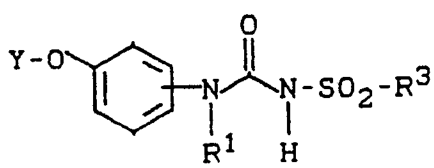

(V)

worin
$R^1$, $R^3$ und Y die oben angegebene Bedeutung haben,
umsetzt,
dann die Schutzgruppe Y nach üblicher Methode abspaltet
und anschließend mit Halogenmethylchinolinen der Formel (VI)

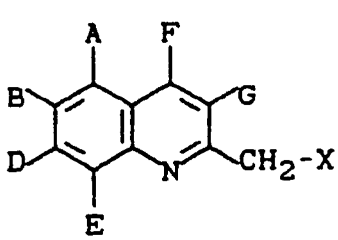

(VI)

in welcher
A, B, D, E, F und G die oben angegebene Bedeutung haben
und

X - für Halogen steht,
verethert,
und im Fall der Verbindungen der Formel (I) mit $R^2 \neq H$ anschließend mit Alkylierungsmitteln alkyliert,
oder indem man

[C] Verbindungen der allgemeinen Formel (VII)

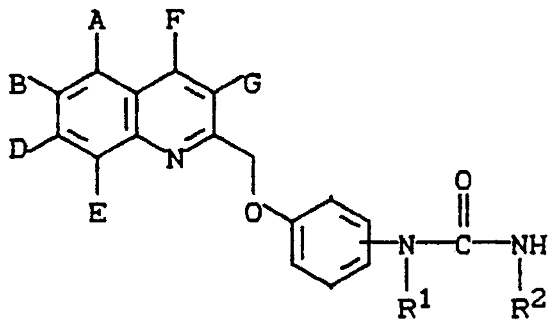

(VII)

in welcher
A, B, D, E, F, G, $R^1$ und $R^2$ die oben angegebene Bedeutung haben,
mit Sulfonsäurehalogeniden der allgemeinen Formel (VIII)
$R^3$-$SO_2$-X (VIII)
in welcher
$R^3$ und X die oben angegebene Bedeutung haben,
in inerten Lösemitteln, gegebenenfalls in Anwesenheit von Basen umsetzt.

6. Arzneimittel enthaltend mindestens einen substituierten (Chinolin-2-yl-methoxy)phenyl-N,N′-sulfonylharnstoff nach Anspruch 1.

7. Verfahren zur Herstellung eines Arzneimittels nach Anspruch 6 dadurch gekennzeichnet, daß man Verbindungen der Formel I nach Anspruch 1 gegebenenfalls mit Hilfe von üblichen Hilfs- und Trägerstoffen in eine geeignete Applikationsform bringt.

8. Verwendung der substituierten (Chinolin-2-yl-methoxy)phenyl-N,N′-sulfonylharnstoffe nach Anspruch 1 zur Bekämpfung von Krankheiten.

9. Verwendung der substituierten (Chinolin-2-yl-methoxy)phenoxy-N,N′-sulfonylharnstoffe nach Anspruch 1 zur Herstellung von Arzneimitteln.

10. Verwendung der substituierten (Chinolin-2-yl-methoxy)phenoxy-N,N′-sulfonylharnstoffe nach Anspruch 1 zur Herstellung von Lipoxygenaseinhibitoren.